# EUROPEAN PATENT SPECIFICATION

(11) **EP 1 698 306 B1**
(45) Date of publication and mention of the grant of the patent: **20.02.2008**
(21) Application number: 05257767.3
(22) Date of filing: 16.12.2005
(51) Int. Cl.: A61F 2/46, A61F 2/34

(54) **Surgical introducer**
Chirurgische Einführungsvorrichtung
Introducteur chirurgical

(30) Priority: 01.03.2005 GB 0504123
(43) Date of publication of application: 06.09.2006
(73) Proprietor: Corin Limited, Cirencester Gloucestershire GL7 1YJ (GB)
(72) Inventor: Collins, Simon Nicholas, Avening,Tetbury GL8 8NT (GB); Climer, Peter James, Beeches Estate, Cirencester GL7 1AT (GB)
(74) Representative: Hocking, Adrian Niall

(56) References cited:
- WO-A-01/10356
- DE-A1- 19 722 923
- DE-U1- 29 823 658
- FR-A- 2 809 305

## Description

The present invention relates to a surgical introducing tool for an acetabular cup, and to a head for the tool which can hold the acetabular cup. The closest prior art is FR-A-2809305, which defines the preamble of claim 1.

It is known from US3859992 and FR2797180 to provide a surgical introducing tool for an acetabular cup which utilises vacuum to hold the cup to the tool.

However, a specific problem with these known arrangements, and also with most other kinds of acetabular cup introducing tools, is that a surgeon will typically tilt the tool in different directions, once the acetabular cup has been placed in the patient's acetabulum, to reposition the cup at the optimum angle. Especially with the introducers using vacuum to hold the cup to the tool, this tilting movement results in the cup breaking free from the tool due to the relative angular displacement of the longitudinal axis of the tool and the polar axis of the cup.

The present invention seeks to provide a solution to this problem.

According to a first aspect of the present invention, there is provided a head for a surgical introducing tool as defined in claim 1.

Preferably and/or optional features of the first aspect of the invention are set forth in claims 2 to 10, inclusive.

According to a second aspect of the invention, there is provided a surgical introducing tool for an acetabular cup, the tool comprising a shaft having a handle at one end, and a head in accordance with the first aspect of the invention at the other end of the shaft.

Preferable and/or optional features of the second aspect of the invention are set forth in claims 12 to 14, inclusive.

According to a third aspect of the invention, there is provided an acetabular cup in combination with a head according to the first aspect of the invention, the cup having one or more formations adapted to accept the or each said retaining element of the head, wherein the or each formation is an elongate slot having closed ends and being formed in a rim of the cup.

Preferable and/or optional features of the third aspect of the invention are set forth in claims 16 to 20, inclusive.

The invention will now be more particularly described, by way of example only, with reference to the accompanying drawings, in which :
Figure 1 is a diagrammatic elevational view of one embodiment of a surgical introducing tool for an acetabular cup, in accordance with the second aspect of the invention, and having a first embodiment of a head in accordance with the first aspect of the invention;
Figure 2 is an enlarged view of the head shown in Figure 1, and an acetabular cup;
Figure 3 is a scrap pictorial view showing exaggerated relative angular displacement between the polar axis of the head and the polar axis of the cup, when engaged;
Figure 4 is a diagrammatic plan view from above of a second embodiment of a head, in accordance with the first aspect of the invention;
Figure 5 is a diagrammatic elevational view of a third embodiment of a head, in accordance with the first aspect of the invention;
Figure 6 is a diagrammatic plan view from below of the head shown in Figure 5;
Figure 7 is a diagrammatic elevational view of a fourth embodiment of a head, in accordance with the first aspect of the invention, shown being offered towards an embodiment of an acetabular cup, in accordance with the third aspect of the invention;
Figure 8 is a view similar to that shown in Figure 7 with the head and acetabular cup engaged;
Figure 9 is a diagrammatic elevational view of a fifth embodiment of a head, in accordance with the first aspect of the invention, shown being offered towards another embodiment of an acetabular cup, in accordance with the third aspect of the invention;
Figure 10 is a view similar to that shown in Figure 9 with the head and acetabular cup engaged;
Figure 11 is a diagrammatic elevational view of a sixth embodiment of a head, in accordance with the first aspect of the invention, shown being offered towards a further embodiment of an acetabular cup, in accordance with the third aspect of the invention;
Figure 12 is a view similar to that shown in Figure 11 with the head and acetabular cup engaged;
Figure 13 is a diagrammatic elevational view of a seventh embodiment of a head, in accordance with the first aspect of the invention, shown being offered towards an embodiment of an acetabular cup, in accordance with the third aspect of the invention; and
Figure 14 is a view similar to that shown in Figure 13 with the head and acetabular cup engaged.

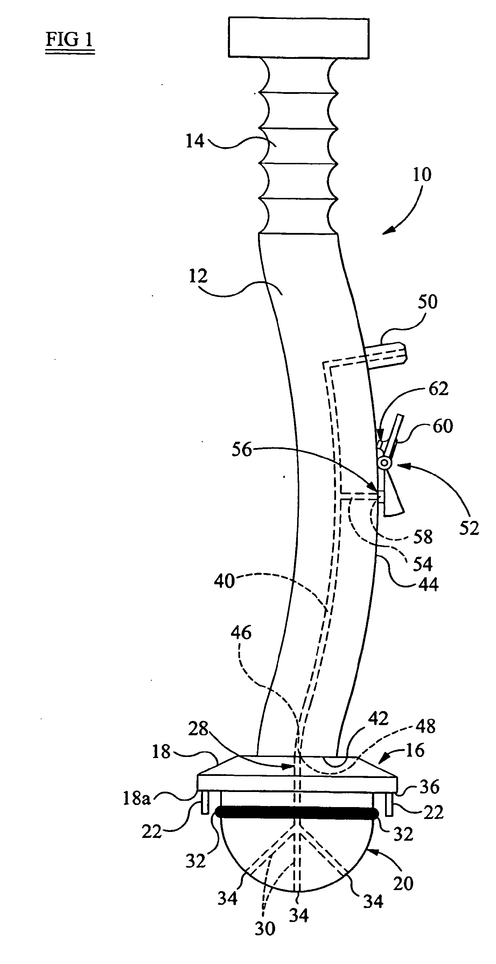

Referring firstly to Figures 1 to 3, there is shown a surgical introducing tool 10 which comprises a shaft 12 having a handle 14 at one end and a head 16 at the other end.

The shaft 12, in this case, is non-rectilinear, being cranked to promote simplified access to a patient's acetabulum during use, particularly during minimal access surgery. However, the shaft 12 may be rectilinear.

The head 16 includes a base plate 18, cup holding element 20 provided centrally on one major surface 18a of the base plate 18, and a plurality of retaining elements 22.

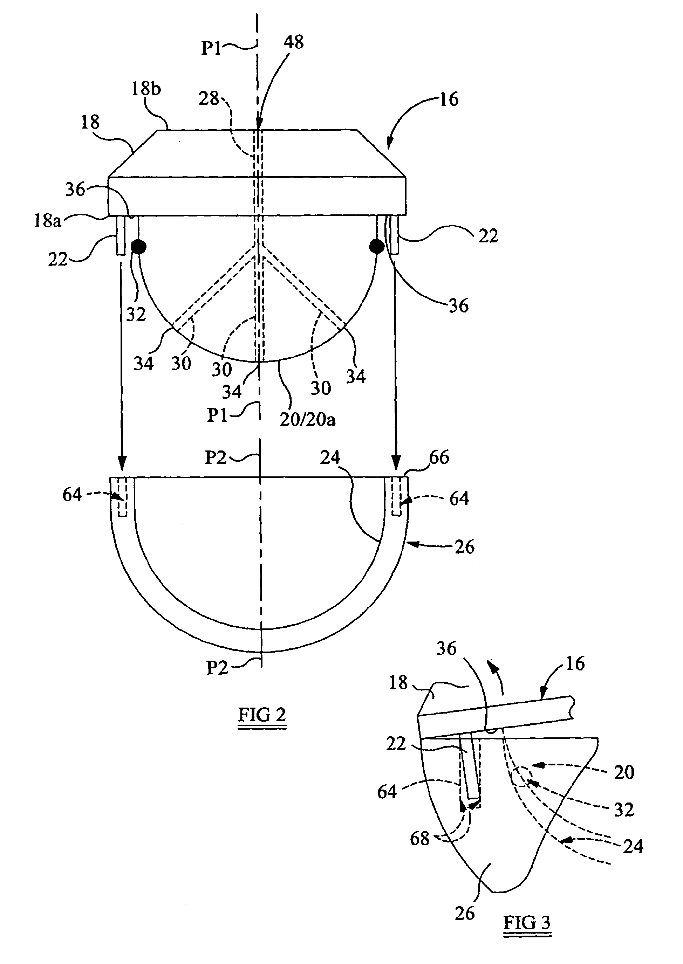

The cup holding element 20 is dimensioned to be a complementary or substantially complementary fit with an articulating inner surface 24 of a known acetabular cup 26, as can be understood from Figure 2. A head air passage 28 passes centrally through the base plate 18, from the other major surface 18b, into the cup holding element 20. The head air passage 28, in the cup holding element 20, branches into three sub-passages 30 which open out on the surface 20a of the cup holding element 20, at positions which are spaced from each other and which are generally remote from the base plate 18.

The cup holding element 20 also includes an O-ring seal 32 which extends around the cup holding element 20 at a position adjacent to the equatorial diameter of the cup 26. The O-ring seal 32 is thus located between the base plate 18 and openings 34 of the air sub-passages 30.

The base plate 18 is dimensioned to overlap the cup holding element 20, thereby providing a flange 36 which extends around the cup holding element 20. The retaining elements 22 project from the flange 36, in spaced relationship to both the cup holding element 20 and each other. In this embodiment, three retaining elements 22 are provided (only two being shown in Figures 1 and 2), and are spaced equiangularly around the cup holding element 20.

The retaining elements 22 project from the flange 36 in parallel with each other, and extend arcuately along the surface of the flange 36.

The head 16 is screwthreadingly engagable, or otherwise modularly connectable, at the base plate 18 with the end of the shaft 12 opposite the handle 14. This allows preoperative and intra-operative changing of the head 16 to one of different dimensions, for example when a different size of acetabular cup is required. However, the head may be permanently connected to the shaft.

The shaft 12 includes an internal air passage 40 which extends partway along the longitudinal extent of the shaft 12. The shaft air passage 40 opens out at the end 42 of the shaft 12 opposite the handle 14, and exits through a side 44 of the shaft 12 partway between the ends. Opening 46 of the shaft air passage 40 in the end 42 of the shaft 12 is positioned to align with opening 48 of the head air passage 28 on the base plate 18 of the head 16. One or more seals (not shown) are provided which, when the head 16 is engaged with the shaft 12, enables the head- and shaft- air passages 28 and 40 to fluid-tightly communicate and thus form a continuous air passage through the tool 10.

The exit of the shaft air passage 40 through the side 44 of the shaft 12 is via a nipple connector 50 integrally formed on the shaft 12. The nipple connector 50 enables connection of a suction line from remote air suction device (not shown).

The shaft 12 also includes a vent arrangement 52 for the shaft air passage 40. In this embodiment, the vent arrangement 52 comprises a branch passage 54 which leads from the shaft air passage 40 and opens out on the side 44 of the shaft 12, and an air-tight cap 56 or stop which covers an opening 58 on the shaft 12 to the branch passage 54. The air-tight cap 56 is provided on a pivotable lever 60 which can be operated via a user's thumb, for example. The lever 60 is urged, typically by a spring 62, to assume a condition whereby the air-tight cap 56 normally seals the opening 58 to the branch passage 54.

In use, and once the head 16 is attached to the shaft 12, an acetabular cup 26 of suitable size is urged onto the cup holding element 20 and retaining elements 22 until rim 66 abuts the base plate 18. The cup holding element 20 is dimensioned so that the O-ring seal 32 forms a fluid-tight interference fit on the articulating inner surface 24 of the acetabular cup 26, and the retaining elements 22 are received as close or tolerance fits in respective openings or slots 64 in the rim 66 of the acetabular cup 26. See Figure 2.

An air line of an air suction device, which is usually available in theatre, is connected to the nipple connector 50 and a vacuum is generated in the space between the articulating inner surface 24 of the acetabular cup 26, the cup holding element 20, and the O-ring seal 32. The acetabular cup 26 is thus securely held by the introducing tool 10.

The acetabular cup 26 is introduced into the patient's body and positioned by impaction in the acetabulum. Once located, the surgeon can move the handle 14 of the tool 10 in a reciprocating manner, in other words angularly displace the longitudinal axis of the tool, to position the cup 26 at the optimum angle. During this tilting movement, and since the acetabular cup 26 will now be held securely in the patient's acetabulum, slight relative angular displacement of polar axis P1 of the head 16 and polar axis P2 of the cup 26 occurs. This results in slight separation or tilting of the head 16 / base plate 18 from the cup 26. The retaining elements 22 of the head 16 consequently move in the openings or slots 64 in the rim 66 of the acetabular cup 26. See Figure 3. Each retaining element 22, when titled due to the slight separation, will tend to bind on the interior surfaces 68 of its respective rim slot 64, thus preventing excessive movement which would otherwise cause the fluid-tight seal generated by the O-ring seal 32 to break. Breakage of the fluid-tight seal causes a loss of vacuum and the acetabular cup 26 to be no longer securely gripped by the tool 10.

Once the surgeon is satisfied that the acetabular cup 26 is securely located in the patient's acetabulum, the pivotable lever 60 on the shaft 12 is operated to lift the air-tight cap 56 and release the vacuum between the cup holding element 20 and the articulating inner surface 24 of the cup 26. The tool 10 can then be easily removed from the cup 26.

Obviously, the closer the tolerance of the fit between the retaining elements 22 and the respective slots 64 in the rim 66 of the acetabular cup 26, the less relative movement impartable therebetween, and the less relative angular displacement between the longitudinal axis of the tool 10 / polar axis P1 of the head 16 and the polar axis P2 of the cup 26.

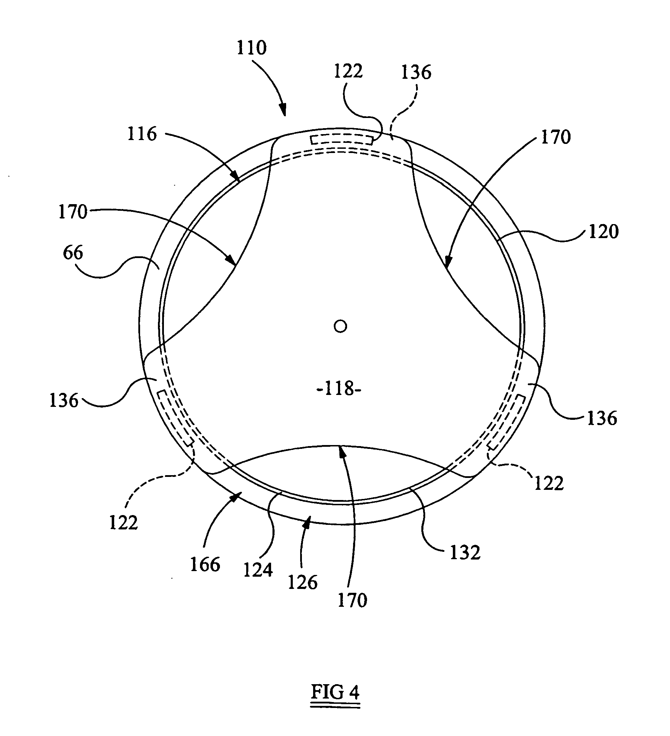

Figure 4 shows a second embodiment of a head 116 of the present invention. Similar references refer to similar parts, and further detailed description is omitted.

The head 116 includes a base plate 118, cup holding element 120, and retaining elements 122, as in the first embodiment. The base plate 118, however, is non-circular, having equi-angularly spaced radially inwardly projecting recesses 170 around the circumference. The number of recesses 170 corresponds to the number of retaining elements 122. In this case, three recesses 170 are provided, which produces three flanges 136 from which the retaining elements 122 extend.

The cup holding element 120 is not correspondingly recessed, due to an interference equatorial or substantially equatorial fit being required between an O-ring seal 132 of the cup holding element 120 and an articulating inner surface 124 of an acetabular cup 126.

The recesses 170 in the base plate 118 allows a user of a tool 110 to see at least a rim 166 of the acetabular cup 126. This is particularly beneficial during introduction and location of the cup 126 into a patient's acetabulum.

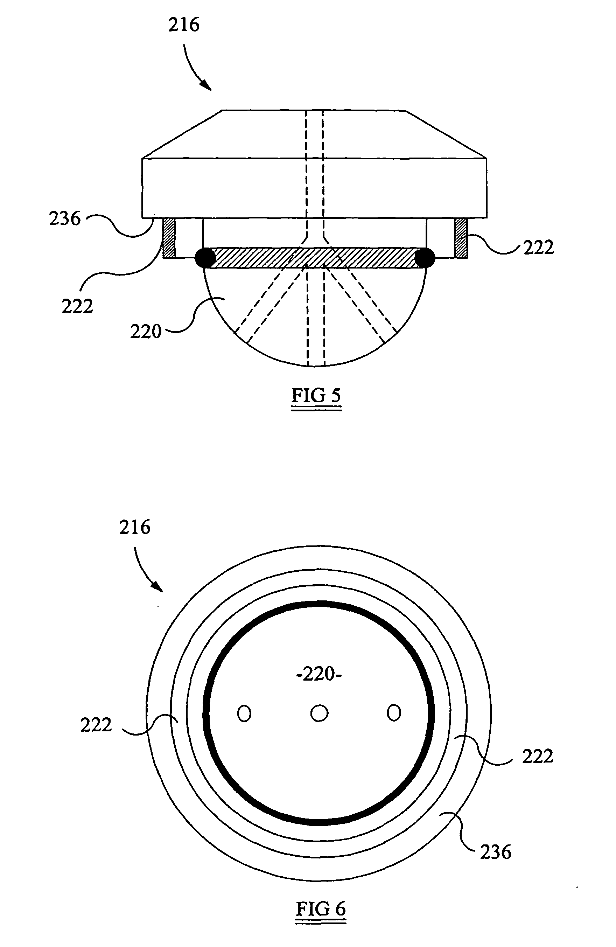

Figures 5 and 6 show a third embodiment of a head 216 of the present invention. Similar references refer to similar parts, and further detailed description is omitted.

In this embodiment, only a single retaining element 222 is provided on a flange 236 of head 216. The retaining element 222 is spaced radially outwards from a cup holding element 220, and extends continuously in a circumferential direction to surround the cup holding element 220.

An acetabular cup (not shown) has a complementarily or substantially complementarily shaped continuous opening, being a slot or channel, formed in a rim of the cup, a continuous inner shoulder formed adjacent to the rim, or a continuous outer shoulder formed adjacent to the rim, depending on the spacing of the retaining element 222 from the cup holding element 220.

In a modification, the retaining element 222 could be a continuous opening, in other words a slot or channel, formed in flange 236 of the head 216, and the acetabular cup therefore has a complementarily or substantially complementarily shaped projection positioned as described above.

In a further modification, two radially spaced concentric continuous retaining elements could be utilised. Complementarily or substantially complementarily shaped formations, being openings or projections, adapted to accept the retaining elements are therefore provided on the acetabular cup.

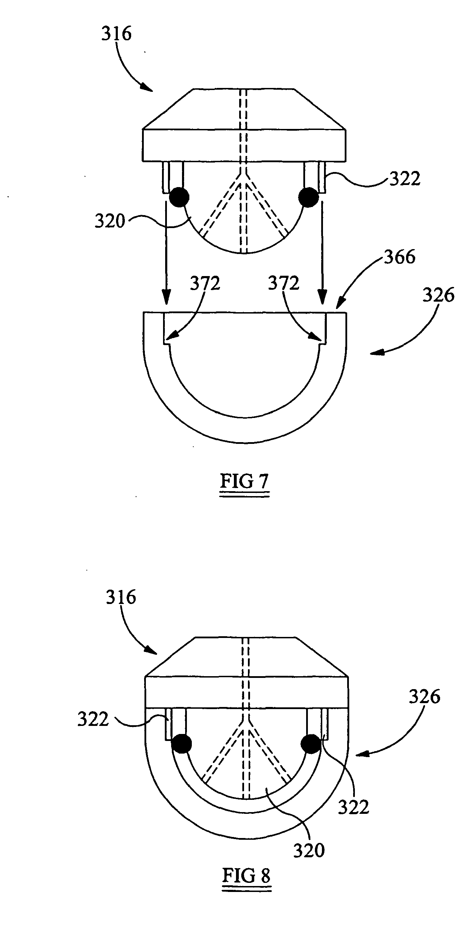

Figures 7 and 8 show a fourth embodiment of a head 316 and an embodiment of an acetabular cup 326. Similar references refer to similar parts, and further detailed description is omitted.

In this embodiment, the acetabular cup 326 includes one or more radially inner shoulders 372 adjacent to rim 366. In use, one or more retaining elements 322 is/are suitably spaced from cup holding element 320 to closely fit the or each shoulder 372.

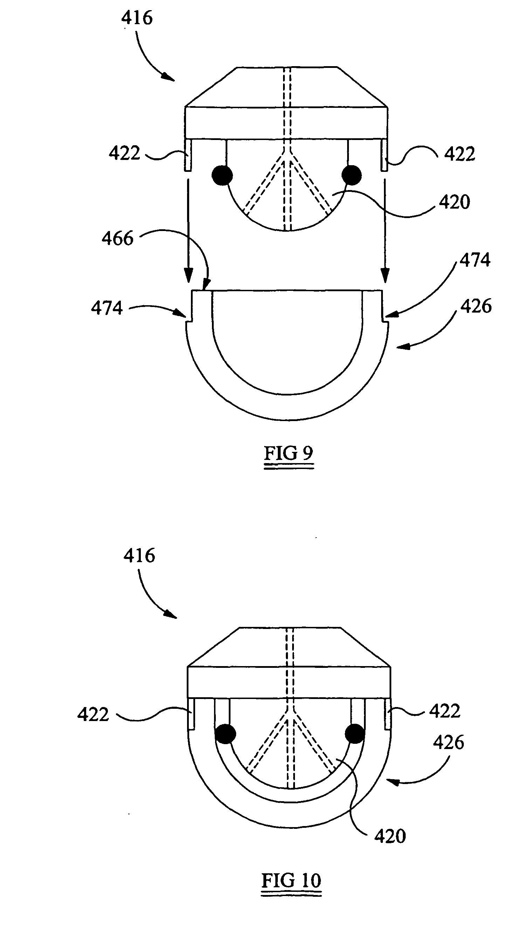

Figures 9 and 10 show a fifth embodiment of a head 416 and another embodiment of an acetabular cup 426. Similar references refer to similar parts, and further detailed description is omitted.

In this embodiment, the acetabular cup 426 includes one or more radially outer shoulders 474 adjacent to rim 466. In use, one or more retaining elements 422 is/are suitably spaced from cup holding element 420 to closely fit the or each outer shoulder 474.

In the fourth and fifth embodiments, it is envisaged that the acetabular cup can, additionally or alternatively, include one or more projections at or adjacent to the inner edge of the rim or the outer edge of the rim, and the or each respective retaining element of the head would therefore be an opening.

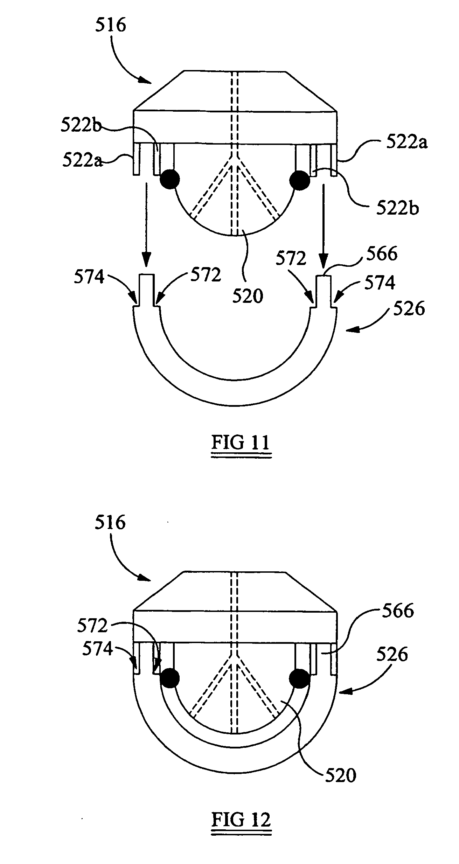

Figures 11 and 12 show a sixth embodiment of a head 516, and another embodiment of an acetabular cup 526. Similar references refer to similar parts, and further detailed description is omitted.

The acetabular cup 526 includes radially inner and outer shoulders 572 and 574 adjacent to rim 566. In use, retaining elements 522a and 522b are suitably spaced from cup holding element 520 to closely fit the shoulders 572 and 574. Retaining element 522a is spaced radially outwardly relative to the retaining element 522b.

Similarly to the earlier embodiments, in a modification, one or more projections could, additionally or alternatively, be included at or adjacent to inner and outer edges of the rim of the acetabular cup, and the respective retaining elements of the head would therefore be openings.

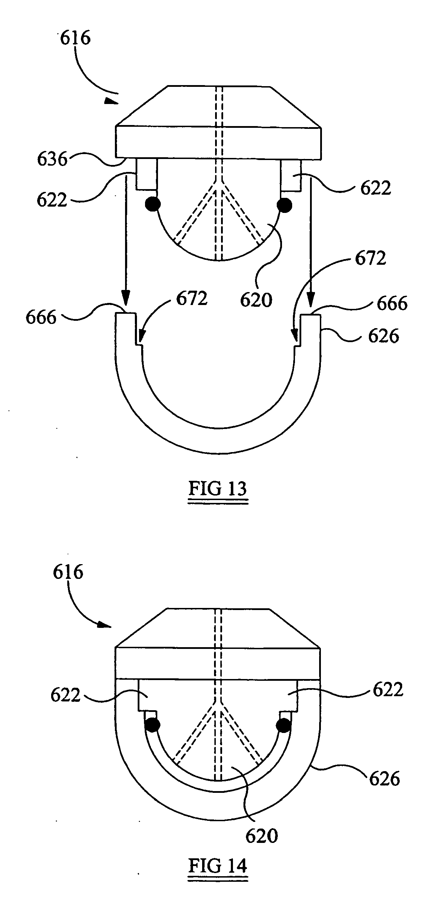

Figures 13 and 14 show a seventh embodiment of a head 616. Similar references refer to similar parts, and further detailed description is omitted.

In this embodiment, one or more retaining elements 622 is/are integrally formed with cup holding element 620 and extend from flange 636. Acetabular cup 626 includes one or more corresponding radially inner shoulders 672 adjacent to rim 666. In use, the or each retaining element 622 radially projects from cup holding element 620 to closely fit the or each inner shoulder 672.

It is envisaged that, in a modification to this embodiment, one or more of the retaining elements could be spaced from the flange of the head.

Except in the case of a continuous retaining element, the retaining elements described above also prevent relative rotational displacement of the head and the cup.

Any suitable number of retaining elements, and therefore corresponding recesses, can be provided. Thus, only a single retaining element may be required.

Although the retaining elements are described as being tongues, the retaining elements can be pins or any other suitably shaped projection. The or each opening, which includes a shoulder and a recess, when in the acetabular cup may be a slot, channel, or any close- or tolerance- fitting complementarily shaped opening.

As mentioned, it is possible that the rim of the acetabular cup can include one or more projections, instead of, or additionally to, the openings or slots. In this case, the retaining elements will be, or include, openings or slots formed in the flange or flanges of the base plate. These corresponding openings or slots will be dimensioned to closely fit the or each projection on the cup.

The retaining element or elements of the head have a close or tolerance fit with the or each corresponding formation on or adjacent to the rim of the cup, in at least a radial direction of the cup holding element.

The cup holding element described above can be any shape providing an interference fit is provided to enable formation of the vacuum seal by the O-ring seal. Regardless of shape, the cup holding element will still inherently have a polar axis.

Although three air sub-passages are suggested in the cup holding element, any suitable number of passages, including a single sub-passage, can be utilised.

Any suitable vent arrangement can be provided.

It is thus possible to provide a head for a surgical introducing tool that more securely holds an acetabular cup during insertion. It is also possible to provide a head for a surgical introducing tool which prevents or limits relative angular displacement of the longitudinal axis of the tool and the polar axis of the acetabular cup.

## Claims

1. A head for a surgical introducing tool (10;110), the head (16; 116;216;316,416-,516;616) comprising:
a cup holding element (20;120;220;320;420;520;620) for releasably holding an acetabular cup (26;126;326;426;526;626), the holding element (20;120;220;320;420;520;620) including a seal (32;132) and an air passage (28) by which the acetabular cup (26; 126;326;426;526;626) can be held to the head (16;116;216;316;416;516;616) by vacuum; and
a retaining element (22;122;222;322;422;522;622) for preventing undesirable release of the acetabular cup (26;126;326;426;526;626) from the holding element (20;120;220;320;420;520;620), the retaining element (22;122;222;322;422;522;622) being positioned radially outwardly relative to the holding element (20;120;220;320;420;520;620), **characterised in that** the retaining element (22,122,222,322,422,522,622) is configured to be received in an opening in or adjacent to the rim of the acetabular cup or to receive a projection on or adjacent to the rim of the acetabular cup,
so that, in use, relative angular displacement of polar axes (P1, P2) of the head (16;116;216;316;416;516;616) and the acetabular cup (26;126;326;426;526;626) is prevented or limited by the retaining element (22;122;222;322;422;522,622) while the cup (26,126;326;426,526;626) is held by the holding element (20;120;220;320;420;520;620), so as to prevent the cup (26;126;326;426;526;626) from being accidentally released from the holding element (20;120;220;320;420;520;620).

2. A head as claimed in claim 1, wherein the head (16;116;216;316;416;516;616) includes a flange (36;136;236;636) on or in which the retaining element (22;122;222;322;422;522;622) is positioned in spaced relationship to the holding element (20;120;220;320;420;520;620).

3. A head as claimed in claim 1, wherein the head (16;116;216;316;416;516;616) includes a flange (36;136;236;636) from which the retaining element (22;122;222;322;422;522;622) extends, and the retaining element (22;122;222;322;422;522;622) is integrally formed with the holding element (20; 120;220;320;420;520;620).

4. A head as claimed in claim 2 or claim 3, wherein the flange (36;636) surrounds the holding element (20;320;420;520;620), and a plurality of said retaining elements (22;322;422;522;622) are positioned on or in the flange (36;636).

5. A head as claimed in claim 2 or claim 3, wherein the flange (236) surrounds the holding element (220), and a single continuous retaining element (222) is positioned on or in the flange (236).

6. A head as claimed in claim 2 or claim 3, wherein a plurality of flanges (136) are provided angularly spaced around the holding element (120), a said retaining element (122) being positioned on or in each flange (136).

7. A head as claimed in any one of the preceding claims, wherein a further retaining element (522a) is positioned radially outwardly relative to the first said retaining element (522b).

8. A head as claimed in any one of the preceding claims, wherein the head includes one or more recesses which project radially inwards.

9. A head as claimed in any one of the preceding claims, wherein the holding element (20;120;220;320;420;520;620) is dimensioned to be an interference fit in at least a portion of the space defined by the articulating inner surface (24;124;224) of the acetabular cup (26;126;326;426;526;626).

10. A head as claimed in any one of the preceding claims, wherein the, each or at least one retaining element (22;122;222;322;422;522;622) is a projection or an opening.

11. A surgical introducing tool (10;110) for an acetabular cup (26;126;326;426;526;626), the tool (10;110) comprising a shaft (12) having a handle (14) at one end, and a head (16;116;216;316;416;516;616) as claimed in any one of the preceding claims at the other end of the shaft.

12. A surgical introducing tool as claimed in claim 11, wherein the head (16;116;216;316;416;516;616) is releasably engaged with the said other end of the shaft (12).

13. A surgical introducing tool as claimed in claim 11 or claim 12, wherein an air passage (40) is formed in the shaft (12), one end of the air passage (40) fluidly communicating with the air passage (28) in the head (16; 116;216;316;416;516;616), and the other end being connectable to an air suction device.

14. A surgical introducing tool as claimed in claim 13, further comprising a vent arrangement (52) for admitting air to the shaft air passage (40).

15. An acetabular cup (26;126;326;426;526;626) in combination with a head (16;116;216;316;416;516;616) for a surgical introducing tool as claimed in any one of claims 1 to 10, the cup having one or more formations (64) adapted to accept the or each said retaining element (22;122;222;322;422;522;622) of the head (16;116;216;316;416;516;616), wherein the or each formation (64) is not an elongate slot having closed ends and being formed in a rim of the cup.

16. An acetabular cup in combination with a head for a surgical introducing tool as claimed in claim 15, wherein the or each formation (64) is an opening formed in a rim (66;166) of the acetabular cup (26;126), a radially inner shoulder (372;572;672) formed adjacent to the rim (366;566;666) of the acetabular cup (326;526;626), and/or a radially outer shoulder (474;574) formed adjacent to the rim (466;566) of the acetabular cup (426;526).

17. An acetabular cup in combination with a head for a surgical introducing tool as claimed in claim 16, wherein a plurality of rim openings, inner shoulders and in combination with a head for a surgical introducing tool or outer shoulders are provided.

18. An acetabular cup in combination with a head for a surgical introducing tool as claimed in claim 16, wherein a single rim opening, inner shoulder and/or outer shoulder is provided.

19. An acetabular cup in combination with a head for a surgical introducing tool as claimed in claim 18, wherein the single rim opening, inner shoulder, and/or outer shoulder is continuous.

20. An acetabular cup in combination with a head for a surgical introducing tool as claimed in claim 15, wherein the, each or at least one formation is a projection formed on the rim of the acetabular cup, the projection being provided on or adjacent to an inner edge of the rim, on or adjacent to an outer edge of the rim, and/or partway between inner and outer edges of the rim.

## Patentansprüche

1. Kopf für ein chirurgisches Fintührungsinstrument (10; 110), wobei der Kopf (16; 116; 216; 316; 416; 516; 616) Folgendes umfasst:
ein Pfannenhalteelement (20; 120; 220; 320; 420; 520; 620) zum lösbaren Halten einer Hüftgelenkpfanne (26; 126; 326; 426; 526; 626) wobei das Halteelement (20; 120; 220; 320; 420; 520; 620) eine Dichtung (32; 132) und einen Luftdurchgang (28) einschließt, wodurch die Hüftgelenkpfanne (26; 126; 326; 426; 526; 626) durch Vakuum an dem Kopf (16; 116; 216; 316; 416; 516; 616) gehalten werden kann, und
ein Rückhalteelement (22; 122; 222; 322; 422; 522; 622) zum Verhindern eines unerwünschten Lösens der Hüftgelenkpfanne (26; 126; 326; 426; 526; 626) von dem Halteelement (20; 120; 220; 320; 420; 520; 620), wobei das Rückhalteelement (22; 122; 222; 322; 422; 522; 622) in Radialrichtung nach außen im Verhältnis zu dem Halteelement (20; 120; 220; 320; 420; 520; 620) angeordnet ist, **dadurch gekennzeichnet, dass** das Rückhalteelement (22; 122; 222; 322; 422; 522; 622) dafür konfiguriert ist, in einer Öffnung in oder benachbart dem Rand der Hüftgelenkpfanne aufgenommen zu werden oder einen Vorsprung an oder benachbart dem Rand der Hüftgelenkpfanne aufzunehmen,
so dass, bei Anwendung, eine relative Winkelverschiebung von Polarachsen (P1, P2) des Kopfes (16; 116; 216; 316; 416; 516; 616) und der Hüftgelenkpfanne (26; 126; 326; 426; 526; 626) durch das Rückhalteelement (22; 122; 222; 322; 422; 522; 622) verhindert oder begrenzt wird, während die Pfanne (26; 126; 326; 426; 526; 626) durch das Halteelement (20; 120; 220; 320; 420; 520; 620) gehalten wird, um so zu verhindern, dass die Pfanne (26; 126; 326; 426; 526; 626) versehentlich von dem Halteelement (20; 120; 220; 320; 420; 520; 620) gelöst wird.

2. Kopfnach Anspruch 1, wobei der Kopf (16; 116; 6; 216; 316; 416; 516; 616) einen Flansch (36; 136; 236; 636) einschließt, an oder in dem das Rückhalteelement (22; 122; 222; 322; 422; 522; 622) in einer Abstandsbeziehung zu dem Halteelement (20; 120; 220; 320; 420; 520; 620) angeordnet ist.

3. Kopf nach Anspruch 1, wobei der Kopf (16; 116; 216; 316; 416; 516; 616) einen Flansch (36; 136; 236; 636) einschließt, von dem aus sich das Rückhalteelement (22; 122; 222; 322; 422; 522; 622) erstreckt, und das Rückhalteelement (22; 122; 222; 322; 422; 522; 622) integral mit dem Halteelement (20; 120; 220; 320; 420; 520; 620) geformt ist.

4. Kopf nach Anspruch 2 oder Anspruch 3, wobei der Flansch (36; 636) das Halteelement (20; 320; 420; 520; 620) umschließt und mehrere der Rückhalteelemente (22; 322; 422; 522; 622) an oder in dem Flansch (36; 636) angeordnet sind.

5. Kopf nach Anspruch 2 oder Anspruch 3, wobei der Flansch (236) das Halteelement (220) umschließt und ein einzelnes durchgehendes Rückhalteelement (222) an oder in dem Flansch (236) angeordnet ist.

6. Kopf nach Anspruch 2 oder Anspruch 3, wobei mehrere Flansche (136) mit Winkelabstand um das Halteelement (120) bereitgestellt werden, wobei ein Rückhalteelement (122) an oder in jedem Flansch (136) angeordnet ist.

7. Kopf nach einem der vorhergehenden Ansprüche, wobei ein weiteres Rückhalteelement (522a) in Radialrichtung nach außen im Verhältnis zu dem ersten Rückhalteelement (522b) angeordnet ist.

8. Kopf nach einem der vorhergehenden Ansprüche, wobei der Kopf eine oder mehrere Aussparungen einschließt, die in Radialrichtung nach innen vorspringen.

9. Kopf nach einem der vorhergehenden Ansprüche, wobei das Halteelement (20; 120; 220; 320; 420; 520; 620) dafür bemessen ist, in wenigstens einem Abschnitt des Raumes, der durch die Gelunkinnenfläche (24; 124; 224) der Hüftgelenkpfanne (26; 126; 326; 426; 526; 626) definiert wird, in Presspassung zu sitzen.

10. Kopf nach einem der vorhergehenden Ansprüche, wobei das, jedes oder wenigstens ein Rückhalteelement (22; 122; 222; 322; 422; 522; 622) ein Vorsprung oder eine Öffnung ist.

11. Chirurgisches Einführungsinstrument (10; 110) für eine Hüftgelenkpfanne (26; 126; 326; 426; 526; 626), wobei das Instrument (10; 110) einen Schaft (12) umfasst, der an dem einen Ende einen Griff (14) und an dem anderen Ende des Schafts einen Kopf (16; 116; 216; 316; 416; 516; 616) nach einem der vorhergehenden Ansprüche hat.

12. Chirurgisches Einführungsinstrument nach Anspruch 11, wobei der Kopf (16; 116; 216; 316; 416; 516; 616) lösbar mit dem anderen Ende des Schafts (12) in Eingriff gebracht ist.

13. Chirurgisches Einführungsinstrument nach Anspruch 11 oder Anspruch 12, wobei ein Luftdurchgang (40) in dem Schaft (12) geformt ist, wobei das eine Ende des Luftdurchgangs (40) in Fluidverbindung mit dem Luftdurchgang (28) in dem Kopf (16; 116; 216; 316; 416; 516; 616) ist und das andere Ende mit einer Luftsaugvorrichtung verbunden werden kann.

14. Chirurgisches Einführungsinstrument nach Anspruch 13, das ferner eine Lüftungsanordnung (52) zum Zuführen von Luft zu dem Schaft-Luftdurchgang (40) umfasst.

15. Hüftgelenkpfanne (26; 126; 326; 426; 526; 626) in Verbindung mit einem Kopf (16; 116; 216; 316; 416; 516; 616) für ein chirurgisches Einführungsinstrument nach einem der Ansprüche 1 bis 10, wobei die Pfanne eine oder mehrere Formationen (64) hat, dafür eingerichtet, das oder jedes Rückhalteelement (22; 122; 222; 322; 422; 522; 622) des Kopfes (16; 116; 216; 316; 416; 516; 616) aufzunehmen, wobei die oder jede Formation (64) nicht ein länglicher Schlitz ist, der geschlossene Enden hat und in einem Rand der Pfanne geformt ist.

16. Hüftgelenkpfanne in Verbindung mit einem Kopf für ein chirurgisches Einfblirungsinstrulment nach Anspruch 15, wobei die oder jede Formation (64) eine in einem Rand (66; 166) der Hüftgelenkpfanne (26, 126) geformte Öffnung, ein in Radialrichtung innerer, angrenzend an den Rand (366; 566; 666) der Hüftgelenkpfanne (326; 526; 626) geformter. Absatz (372, 572; 672) und/oder ein in Radialrichtung äußerer, angrenzend an den Rand (466; 566) der Hüftgelentkpfanne (426, 526) geformter, Absatz (474, 574) ist.

17. Hüftgelenkpfanne in Verbindung mit einem Kopf für ein chirurgisches Einführungsinstrument nach Anspruch 16, wobei mehrere Randöffnungen, innere Absätze und/oder äußere Absätze bereitgestellt werden.

18. Hüftgelenkpfanne in Verbindung mit einem Kopf für ein chirurgisches Einführungsinstrument nach Anspruch 16, wobei eine einzige Randöffnung, ein einziger innerer Absatz und/oder äußerer Absatz bereitgestellt wird.

19. Hüftgelenkpfanne in Verbindung mit einem Kopf für ein chirurgisches Einführungsinstrument nach Anspruch 18, wobei die einzige Randöffnung, der einzige innere Absatz und/oder äußere Absatz durchgehend ist.

20. Hüftgelenkpfanne in Verbindung mit einem Kopf für ein chirurgisches Einführungsinstrument nach Anspruch 15, wobei die oder jede oder wenigstens eine Formation ein an dem Rand der Hüftgelenkpfanne geformter Vorsprung ist, wobei der Vorsprung an oder benachbart einer Innenkante des Randes, an oder benachbart einer Außenkante des Randes und/oder auf halbem Wege zwischen der Innen- und der Außenkante des Randes bereitgestellt wird.

## Revendications

1. Tête pour un outil d'introduction chirurgical (10; 110), la tête (16 ; 116 ; 216 ; 316 ; 4 16 ; 516 ; 616) comprenant :
un élément de retenue du cotyle (20 ; 120 ; 220 ; 320 ; 420 ; 520 ; 620) pour retenir de manière amovible un cotyle prothétique (26 ; 126 ; 326 ; 426 ; 526 ; 626), l'élément de retenue (20 ; 120 ; 220 ; 320 ; 420 ; 520 ; 620) englobant un joint (32 ; 132) et un passage d'air (28), permettant de retenir le cotyle prothétique (26 ; 126 ; 326 ; 426 ; 526 ; 626) sur la tête (16 ; 116 ; 216 ; 316 ; 416 ; 516 ; 616) par le vide ; et
un élément de retenue (22 ; 122 ; 222 ; 322 ; 422 ; 522 ; 622) pour empêcher un dégagement indésirable du cotyle prothétique (26 ; 126 ; 326 ; 426 ; 526 ; 626) de l'élément de retenue (20 ; 120 ; 220 ; 320 ; 420 ; 520 ; 620), l'élément de retenue (22 ; 122 ; 222 ; 322 ; 422 ; 522 ; 622) étant positionné radialement vers l'extérieur de l'élément de retenue (20 ; 120 ; 220 ; 320 ; 420 ; 520 ; 620), **caractérisée en ce que** l'élément de retenue (22 ; 122 ; 222 ; 322 ; 422 ; 522; 622) est configuré de sorte à être reçu dans une ouverture dans le rebord du cotyle prothétique ou adjacente celui-ci, ou à recevoir une saillie sur le rebord du cotyle prothétique ou adjacente à celui-ci ;
de sorte qu'en service, un déplacement angulaire relatif des axes polaires (P1, P2) de la tête (16 ; 116 ; 216 ; 316 ; 416 ; 516 ; 616) et du cotyle prothétique (26 ; 126 ; 326 ; 426 ; 526 ; 626) est empêche ou limité par l'élément de retenue (22; 122 ; 222 ; 322 ; 422 ; 522 ; 622), le cotyle (26 ; 126 ; 326 ; 426 ; 526 ; 626) étant retenu par l'élément de retenue (20; 120; 220; 320; 420; 520; 620), de sorte à empêcher un dégagement accidentel du cotyle (26; 126; 326; 426 ; 526; 626) de l'élément de retenue (20; 120; 220; 320 ; 420; 520 ; 620).

2. Tête selon la revendication 1, dans laquelle la tête (16; 116; 216; 316; 416; 516; 616) englobe une bride (36; 136; 236; 636) sur ou dans laquelle est positionné l'élément de retenue (22; 122; 222; 322; 422; 522 ; 622), d'une manière espacée par rapport à l'élément de retenue (20; 120; 220; 320; 420; 520; 620).

3. Tête selon la revendication 1, dans laquelle la tête (16; 116; 216; 316; 416; 516 ; 616) englobe une bride (36 ; 136; 236; 636), à partir de laquelle s'étend l'élément de retenue (22 ; 122; 222; 322 ; 422; 522; 622), l'élément de retenue (22; 122; 222; 322 ; 422 ; 522; 622) étant formé d'une seule pièce avec l'élément de retenue (20 ; 120; 220; 320 ; 420; 520 ; 620).

4. Tête selon les revendications 2 ou 3, dans laquelle la bride (36 , 636) entoure l'élément de retenue (20; 320 ; 420; 520 ; 620), plusieurs desdits éléments de retenue (22 ; 322 ; 422 ; 522 ; 622) étant positionnés sur ou dans la bride (36 ; 636).

5. Tête selon les revendications 2 ou 3, dans laquelle la bride (236) entoure l'élément de retenue (220), un seul élément de retenue continu (222) étant positionné sur ou dans la bride (236).

6. Tête selon les revendications 2 ou 3, dans laquelle plusieurs brides (136) sont espacées angulairement autour de l'élément de retenue (120), un dit élément de retenue (122) étant positionné sur ou dans chaque bride (136).

7. Tête selon l'une quelconque des revendications précédentes, dans laquelle un élément de retenue additionnel (522a) est positionné radialement vers l'extérieur dudit premier élément de retenue (522b).

8. Tête selon l'une quelconque des revendications précédentes, dans laquelle la tête englobe un ou plusieurs évidements débordant radialement vers l'intérieur.

9. Tête selon l'une quelconque des revendications précédentes, dans laquelle l'élément de retenue (20 ; 120 ; 220 ; 320 ; 420 ; 520 ; 620) est dimensionné de sorte à être ajusté par serrage dans au moins une partie de l'espace défini par la surface interne articulée (24 ; 124 ; 224) du cotyle prothétique (26; 126; 326 ; 426 ; 526 ; 626).

10. Tête selon l'une quelconque des revendications précédentes, dans laquelle l'élément de retenue, chaque élément de retenue ou au moins un élément de retenue (22 ; 122 ; 222 ; 322 ; 422 ; 522 ; 622) est constitué par une saillie ou une ouverture.

11. Outil d'introduction chirurgical (10; 110) pour un cotyle prothétique (26 ; 126 ; 326 ; 426 ; 526; 626), l'outil (0; 110) comprenant un arbre (12), comportant un manche (14) au niveau d'une extrémité, et une tête (16 ; 116; 216 ; 316 ; 416; 516; 616) selon l'une quelconque des revendications précédentes au niveau de l'autre extrémité de l'arbre.

12. Outil d'introduction chirurgical selon la revendication 11, dans lequel la tête (16; 116; 216 ; 316; 416 ; 516; 616) est engagée de manière amovible dans ladite autre extrémité de l'arbre (12).

13. Outil d'introduction chirurgical selon les revendications 11 ou 12, dans lequel un passage d'air (40) est formé dans l'arbre (12), une extrémité du passage d'air (40) étant en communication de fluide avec le passage d'air (28) dans la tête (16 ; 116 ; 216 ; 316 ; 416 ; 516 ; 616), l'autre extrémité pouvant être connectée à un dispositif d'aspiration d'air.

14. Outil d'introduction chirurgical selon la revendication 13, comprenant en outre un dispositif d'évent (52) pour admettre de l'air dans le passage d'air de l'arbre (40).

15. Cotyle prothétique (26 ; 126 ; 326 ; 426 ; 526 ; 626), en combinaison avec une tête (16 ; 116 ; 216 ; 316 ; 416 ; 516 ; 616) selon l'une quelconque des revendications 1 à 10, le cotyle comportant une ou plusieurs structures (64) adaptées pour recevoir le ou chaque dit élément de retenue (22 ; 122 ; 222 ; 322 ; 422 ; 522 ; 622) de la tête (16 ; 116 ; 216 ; 316 ; 416 ; 516 ; 616), la ou chaque structure (64) n'étant pas constituée par une fente allongée comportant des extrémités étroites et formée dans un rebord du cotyle.

16. Cotyle prothétique, en combinaison avec une tête pour un outil d'introduction chirurgical, selon la revendication 15, dans lequel la structure ou chaque structure (64) est constituée par une ouverture formée dans un rebord (66, 166) du cotyle prothétique (26 ; 126), un épaulement radialement interne (372 ; 572 ; 672) formé près du rebord (366; 566 ; 666) du cotyle prothétique (326; 526 ; 626), et/u un épaulement radialement externe (474 ; 574) formé près du rebord (466 ; 566) du cotyle prothétique (426 ; 526).

17. Cotyle prothétique,en combinaison avec une tête pour un outil d'introduction chirurgical, selon la revendication 16, comportant plusieurs ouvertures de rebord, plusieurs épaulements internes et/ou épaulements externes.

18. Cotyle prothétique, en combinaison avec une tête pour un outil d'introduction chirurgical, selon la revendication 16, comportant une seule ouverture de rebord, un seul épaulement interne/et ou épaulement externe.

19. Cotyle prothétique, en combinaison avec une tête pour un outil d'introduction chirurgical, selon la revendication 18, dans lequel la seule ouverture de rebord, le seul épaulement interne et/ou épaulement externe est continu.

20. Cotyle prothétique, en combinaison avec une tête pour un outil d'introduction chirurgical, selon la revendication 15, dans lequel chaque structure ou au moins une structure est constituée par une saillie formée sur le rebord du cotyle prothétique, la saillie étant agencée sur un bord interne du rebord ou en un point adjacent à celui-ci, sur un bord externe du rebord ou en un point adjacent à celui-ci et/ou en partie entre les bords interne et externe du rebord.
